# EUROPEAN PATENT APPLICATION

(11) **EP 4 549 014 A1**
(43) Date of publication of application: **07.05.2025**
(21) Application number: 23830213.7
(22) Date of filing: 27.06.2023
(51) Int. Cl.: B01J 29/12

(54) **SILICON-ALUMINUM MOLECULAR SIEVE-HYDROGENATION METAL COMPONENT-ALUMINA COMPLEX, AND PREPARATION AND USE THEREOF**

(30) Priority: 28.06.2022 CN 202210742780
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: WANG, Wennian, Shanghai 201208 (CN); GAO, Huanxin, Shanghai 201208 (CN); JIA, Yinjuan, Shanghai 201208 (CN); XU, Ming, Shanghai 201208 (CN); LIU, Yuanlin, Shanghai 201208 (CN); YAO, Hui, Shanghai 201208 (CN); WEI, Yilun, Shanghai 201208 (CN); YOU, Dandan, Shanghai 201208 (CN)
(74) Representative: karo IP
(86) International application number: PCT/CN2023/102547
(87) International publication number: WO 2024/002036

(57) **Abstract**

Disclosed are a composite of aluminosilicate zeolite-hydrogenation metal component-alumina, and preparation and use thereof, wherein the composite comprises an aluminosilicate zeolite, alumina and a hydrogenation metal component, wherein at least portion of the aluminosilicate zeolite in the composite is chemically bonded to at least portion of the alumina, such that the composite has an infrared spectrum exhibiting a characteristic peak within a range of 860-900 cm⁻¹. When the hydroalkylation catalyst based on the composite is used in the hydroalkylation reaction of benzene to prepare cyclohexylbenzene, the excessive hydrogenation of benzene caused by too long diffusion path can be avoided, the selectivity of cyclohexane as a byproduct can be reduced, the utilization rate of benzene can be improved, and the energy consumption of reaction and separation can be reduced.

## Description

### Technical Field

The present application relates to the technical field of catalysts, and specifically to an aluminosilicate zeolite-hydrogenation metal component-alumina composite suitable for use as a catalytically active component, and preparation and use thereof.

### Background Art

Cyclohexylbenzene is an important chemical intermediate, which can be used for preparing phenol and cyclohexanone by oxidation, and can also be used as an additive for electrolyte of lithium-ion secondary battery, which has the function of preventing overcharge. Owing to its relatively high cetane number, it can also be used as a blending component for cetane number of diesel oil.

WO001244A discloses a method for synthesizing cyclohexylbenzene by catalyzing the alkylation of benzene and cyclohexene using solid acid Y zeolite as a catalyst, but the yield of cyclohexylbenzene in this process is less than 90%. CN104513122A discloses a method for synthesizing cyclohexylbenzene by liquid-phase alkylation of benzene and cyclohexene, which comprises alkylating a mixture of cyclohexene and cyclohexane with benzene by using MWW zeolite as a catalyst to obtain cyclohexylbenzene, with a high cyclohexene conversion rate and a cyclohexylbenzene yield of more than 90%.

CN108435234A discloses a method for preparing cyclohexylbenzene by catalyzing the alkylation of benzene and cyclohexene using heteropolyacid as a catalyst, with a cyclohexylbenzene yield reaching 96% and a cyclohexene conversion rate of 86% after the catalyst has been reused for 10 times. Although the yield of cyclohexylbenzene in the method of alkylating benzene and cyclohexene is relatively high, the cost of cyclohexene is relatively high as cyclohexene in industry comes from the selective hydrogenation of benzene and the separation of cyclohexene and benzene is difficult due to their close boiling points.

Benzene hydroalkylation technology has the characteristics of simple and easy-to-obtain raw materials and short process, and thus is another ideal choice for the production of cyclohexylbenzene. US4094918 discloses a four-component catalyst with 13X zeolite as a carrier, which exhibits excellent hydroalkylation performance. Since then, hydroalkylation catalysts with zeolite as the alkylation component have been extensively developed. US5053571, US5146024, US6037513 and CN103261126A disclose the use of β zeolite, X or Y zeolite and MCM-22 zeolite, respectively, as the alkylation component in hydroalkylation reactions. Although the reaction comprises two steps: hydrogenation of benzene to produce cyclohexene and alkylation of cyclohexene with benzene, it is completed on the same catalyst. Therefore, once the coupling performance of the two reactions of hydrogenation and alkylation is improved, the by-products of these reactions can be reduced well.

In the prior art, the zeolite and the carrier used in the hydroalkylation catalyst are independent from each other, so that the hydrogenation and alkylation processes conducted over the catalyst cannot be well coupled, resulting in excessive hydrogenation of benzene and generation of by-product, cyclohexane, at a higher content. On one hand, cyclohexane cannot be further utilized in the system, resulting in a waste of benzene resources; on the other hand, the boiling points of benzene and cyclohexane are very close, making separation difficult, resulting in increased energy consumption of the reaction.

Accordingly, there remains a need in the art for a catalyst that can achieve hydroalkylation reaction of aromatics with high conversion rate and selectivity.

### Summary of Invention

Aiming at overcoming the defects of the prior art, the present application provides an aluminosilicate zeolite-hydrogenation metal component-alumina composite, and preparation and use thereof. The composite is particularly suitable as a catalytically active component of a hydroalkylation catalyst for catalyzing hydroalkylation reaction of aromatics.

In order to achieve the above object, in one aspect, the present application provides an aluminosilicate zeolite-hydrogenation metal component-alumina composite, comprising an aluminosilicate zeolite, alumina and a hydrogenation metal component, wherein at least portion of the aluminosilicate zeolite in the composite is chemically bonded to at least portion of the alumina, such that the composite has an infrared spectrum exhibiting a characteristic peak within a range of 860-900 cm⁻¹.

In another aspect, there is provided a method for preparing the aluminosilicate zeolite-hydrogenation metal component-alumina composite of the present application, comprising steps of:
1) subjecting hydrated alumina to a first calcination, a treatment with an aqueous ammonium salt solution, and a second calcination in sequence to obtain an activated alumina substrate;
2) loading a hydrogenation metal component on the activated alumina substrate to obtain a modified activated alumina substrate; and
3) using the modified activated alumina substrate as a solid aluminum source and synthesizing an aluminosilicate zeolite by hydrothermal crystallization to obtain the composite.

In yet another aspect, the present application provides an aluminosilicate zeolite based catalyst comprising a binder and the aluminosilicate zeolite-hydrogenation metal component-alumina composite of the present application, wherein the catalyst has an aluminum content (calculated as alumina) of 10-85 wt%, a silicon content (calculated as silica) of 10-85 wt%, and a hydrogenation metal component content (calculated as metal) of 0.01-5 wt%, based on the total mass of the catalyst.

In yet another aspect, there is provided a method for preparing an aluminosilicate zeolite based catalyst, comprising steps of:
I) providing the aluminosilicate zeolite-hydrogenation metal component-alumina composite of the present application or preparing the aluminosilicate zeolite-hydrogenation metal component-alumina composite according to the method of the present application for preparing the aluminosilicate zeolite-hydrogenation metal component-alumina composite; and
II) kneading and molding the aluminosilicate zeolite-hydrogenation metal component-alumina composite with a binder source, followed by calcination and optionally activation, to obtain the catalyst.

In yet another aspect, there is provided an aluminosilicate zeolite based catalyst prepared according to the method of the present application for preparing an aluminosilicate zeolite based catalyst.

In yet another aspect, there is provided use of the aluminosilicate zeolite based catalyst of the present application in hydroalkylation reaction of aromatics, comprising contacting and reacting a feedstock of aromatics and an alkylating agent with the aluminosilicate zeolite based catalyst in the presence of hydrogen.

In still another aspect, the present application provides a method for synthesizing cyclohexylbenzene, comprising steps of: contacting and reacting benzene with the aluminosilicate zeolite based catalyst of the present application in the presence of hydrogen to obtain cyclohexylbenzene.

Compared with the prior art, the aluminosilicate zeolite based catalyst using the aluminosilicate zeolite-hydrogenation metal component-alumina composite of the present application as the catalytically active component has good catalytic activity and selectivity when being used in the hydroalkylation reaction of aromatics, particularly can avoid excessive hydrogenation of benzene, reduce the selectivity of cyclohexane as a byproduct, improve the utilization rate of benzene and reduce the energy consumption of reaction and separation when being used in the hydroalkylation reaction of benzene to prepare cyclohexylbenzene.

Additional features and advantages of the present application will be set forth in detail in the detailed description which follows.

### Brief Description of the Drawing

The accompanying drawings, which are used to provide a further understanding of the present application and constitute a part of this specification, illustrate embodiments of the present application together with the detailed description which follows, but do not constitute a limitation to the present application. In the drawings:
FIG. 1 is a graph showing the long-term run results of the aluminosilicate zeolite based catalysts obtained in Example 1 of the present application and Comparative Example 1;
FIG. 2 shows an infrared spectrum of the Y-Ru/Al₂O₃ composite obtained in Example 1; and
FIG. 3 shows an infrared spectrum of the Y-Ru/Al₂O₃ composite obtained in Comparative Example 1.

### Detailed Description of Invention

The following provides a detailed explanation of specific embodiments of the present application in conjunction with the accompanying drawings. It should be understood that the specific embodiments described herein are only intended to illustrate and explain the present application, and are not intended to limit the present application.

Any specific numerical value disclosed herein (including endpoints of ranges of numerical values) is not to be limited to the precise value of that numerical value, and is to be understood to also encompass values close to the precise value, for example, all possible values within ±5% of the precise value. Also, for the disclosed ranges of numerical values, one or more new ranges of numerical values can be obtained by any combination between the endpoint values of the range, between the endpoint values and specific point values within the range, and between each specific point values, and such new ranges of numerical values should also be considered to be specifically disclosed herein.

Unless otherwise indicated, the terms used herein have the same meanings as commonly understood by those skilled in the art. If a term is defined herein and its definition is different from the common understanding in the art, the definition herein shall prevail.

In the present application, the so-called "hydrogenation metal component" refers to a metal, a metal oxide, other metal compound, or mixtures thereof having hydrogenation activity.

In the present application, the composition of the aluminosilicate zeolite-hydrogenation metal component-alumina composite and the aluminosilicate zeolite based catalyst is determined by X-ray fluorescence (XRF) analysis, and is consistent with the addition ratio of related raw materials during preparation.

In the present application, the zeolite crystallinity of the aluminosilicate zeolite-hydrogenation metal component-alumina composite and the aluminosilicate zeolite based catalyst is measured by X-ray diffraction (XRD) method, and the sample is dried at 120°C for 2 hours before testing. The crystallinity of the sample is calculated from the peak area of the diffraction peak between 2 theta 5-40 ° on the XRD spectrum.

In the present application, the surface hydrogenation metal percentage content of the aluminosilicate zeolite-hydrogenation metal component-alumina composite is determined by X-ray photoelectron spectroscopy (XPS) method; the total hydrogenation metal percentage content of the composite is determined by X-ray fluorescence spectroscopy (XRF) method.

In the present application, the given pressures are gauge pressures, unless otherwise specified.

In the present application, anything or matters not mentioned are directly applicable to aspects known in the art without any changes except for the contents explicitly stated. Moreover, any embodiment described herein may be freely combined with one or more other embodiments described herein, and the technical solutions or concepts formed thereby are deemed to be part of the original disclosure or original description of the present application, and should not be considered as new matters not disclosed or contemplated herein, unless such combination is considered clearly unreasonable by those skilled in the art.

All patent and non-patent documents mentioned herein, including but not limited to textbooks and journal articles and the like, are incorporated herein by reference in their entirety.

As described above, in the first aspect, the present application provides an aluminosilicate zeolite-hydrogenation metal component-alumina composite, comprising an aluminosilicate zeolite, alumina and a hydrogenation metal component, wherein at least portion of the aluminosilicate zeolite in the composite is chemically bonded to at least portion of the alumina, such that the composite has an infrared spectrum exhibiting a characteristic peak within a range of 860-900 cm⁻¹.

In a preferred embodiment, the aluminosilicate zeolite is selected from zeolites having a ten- or twelve-membered ring pore structure, or combinations thereof, more preferably from β, Y, MCM-22, PSH-3, SSZ-25, MCM-49, MCM-56 zeolites, or combinations thereof, and particularly preferably from β, Y, MCM-22 zeolites, or combinations thereof.

In the present application, there is no particular limitation on the type of hydrogenation active metal in the hydrogenation metal component, and it can be various hydrogenation active metals conventionally used in the art. In a preferred embodiment, the hydrogenation metal component comprises hydrogenation active metal(s) selected from metals from Group VIB and Group VIII of the Periodic Table, or combinations thereof, more preferably from Ru, Pd, Pt, Ni, Co, Mo, W, or combinations thereof, and particularly preferably from Pd, Ru, Ni, or combinations thereof. The adoption of the above-mentioned preferable hydrogenation active metal is beneficial to improving the comprehensive performance of the catalyst.

In a preferred embodiment, the composite has a zeolite crystallinity of 50-75%, preferably 50-70%, as detected by XRD.

In a preferred embodiment, the composite has a silicon content (calculated as silica) of 5-65 wt%, preferably 15-50 wt%, an aluminum content (calculated as alumina) of 30-94.99 wt%, preferably 50-85% and a hydrogenation metal component content (calculated as metal) of 0.01-5 wt%, preferably 0.1-3 wt%, based on the total mass of the composite.

In a preferred embodiment, the surface hydrogenation metal percentage content of the composite as measured by XPS method accounts for 0.1-25%, preferably 5-20% of the total hydrogenation metal percentage content of the composite as measured by XRF method.

In a preferred embodiment, the composite of the present application is prepared by a method comprising steps of:
1) subjecting hydrated alumina to a first calcination, a treatment with an aqueous ammonium salt solution, and a second calcination in sequence to obtain an activated alumina substrate;
2) loading a hydrogenation metal component on the activated alumina substrate to obtain a modified activated alumina substrate; and
3) using the modified activated alumina substrate as a solid aluminum source and synthesizing an aluminosilicate zeolite by hydrothermal crystallization to obtain the composite.

Further preferably, each feature of the steps 1) to 3) is as described in the second aspect below.

Without being bound to any specific theory, it is believed that in the aluminosilicate zeolite-hydrogenation metal component-alumina composite of the present application, most of the hydrogenation active metal is sandwiched between the zeolite and the alumina, rather than being dispersed on the outer surface of the zeolite, which is more conducive to improving the coupling of hydrogenation and alkylation reactions, and especially in the hydroalkylation reaction of benzene to produce cyclohexylbenzene, side reactions such as excessive hydrogenation of benzene caused by too long diffusion path can be avoided, thereby reducing the selectivity of cyclohexane as a byproduct, improving the utilization rate of benzene and reducing the energy consumption for reaction and separation.

In the second aspect, the present application provides a method for preparing an aluminosilicate zeolite-hydrogenation metal component-alumina composite, comprising steps of:
1) subjecting hydrated alumina (such as pseudo-boehmite) to a first calcination, a treatment with an aqueous ammonium salt solution, and a second calcination in sequence to obtain an activated alumina substrate;
2) loading a hydrogenation metal component on the activated alumina substrate to obtain a modified activated alumina substrate; and
3) using the modified activated alumina substrate as a solid aluminum source and synthesizing an aluminosilicate zeolite by hydrothermal crystallization to obtain the composite.

Without being bound to any specific theory, it is believed that in the method for preparing the aluminosilicate zeolite-hydrogenation metal component-alumina composite of the present application, the modified activated alumina loaded with the hydrogenation metal component is used as a solid aluminum source, and the aluminosilicate zeolite is synthesized by hydrothermal crystallization, such that the zeolite is formed on the surface of the alumina loaded with the hydrogenation active metal on the surface, such that most of the hydrogenation active metal is sandwiched between the zeolite and the alumina, which is more conducive to improving the coupling of hydrogenation and alkylation reactions, especially in the reaction of benzene hydroalkylation to prepare cyclohexylbenzene. This can avoid excessive hydrogenation of benzene caused by too long diffusion path, thereby reducing the selectivity of cyclohexane as a byproduct, improving the utilization rate of benzene, and reducing the energy consumption of reaction and separation.

According to the present application, suitable hydrated alumina includes, but is not limited to, pseudo-boehmite, alumina trihydrate, boehmite, amorphous aluminum hydroxide, or mixtures thereof, preferably pseudo-boehmite.

In a preferred embodiment, conditions of the first calcination and the second calcination in step 1) each independently include: a calcination temperature of 500-650°C, preferably 550-600°C and/or a calcination time of 0.5-5 hours, preferably 2-4.5 hours.

According to the present application, the "treatment with an aqueous ammonium salt solution" is a treatment with an acidic ammonium salt solution, such as an ammonium chloride solution, an ammonium nitrate solution, an ammonium sulfate solution, or the like. In a preferred embodiment, conditions of the treatment with an aqueous ammonium salt solution in step 1) include: a mass ratio of the hydrated alumina (based on the weight after calcination at 550°C for 5 hours) to the ammonium salt to water of 1: 1-15: 1-15, preferably 1: 1-2: 1-5; an exchange temperature of 25-100°C, preferably 60-90°C; and/or an exchange time of 0.5-5 hours, preferably 1-2 hours.

Without being bound to any specific theory, it is believed that in the method of the present application, by using hydrated alumina as the raw material and subjecting it to calcination, treatment with an aqueous ammonium salt solution and secondary calcination treatment to synthesize the activated alumina substrate which is used as a raw material for preparing the zeolite, the crystallinity of the zeolite can be improved, thereby being more conducive to the synergistic cooperation between the obtained zeolite and the metal component, which together can improve the performance of the catalyst using the zeolite as the active component, and especially in the reaction of preparing cyclohexylbenzene by hydroalkylation of benzene, the selectivity of cyclohexane as a byproduct can be better reduced.

In a preferred embodiment, the hydrogenation metal component comprises hydrogenation active metal(s) selected from metals of Group VIB and Group VIII of the Periodic Table, or combinations thereof, more preferably from Ru, Pd, Pt, Ni, Co, Mo, W, or combinations thereof, and particularly preferably from Pd, Ru, Ni, or combinations thereof.

In a preferred embodiment, the loading in step (2) is achieved by impregnation, preferably by equal-volume impregnation. Further preferably, the loading in step (2) is carried out by: contacting the activated alumina substrate with an aqueous solution containing a soluble salt of the hydrogenation active metal at a contact temperature of 0-50°C for a contact time of 0.5-12 hours, wherein the hydrogenation active metal is as described above and will not be described here.

In a preferred embodiment, the step (3) is carried out by: reacting the modified activated alumina substrate, a silicon source, an alkali source, optionally a structure directing agent or template agent, and water under hydrothermal crystallization conditions to obtain the composite.

In a further preferred embodiment, the hydrothermal crystallization conditions include: a hydrothermal crystallization temperature of 80-250°C, preferably 100-180°C, and/or a hydrothermal crystallization time of 20-60 hours, preferably 28-48 hours. In a further preferred embodiment, different reaction conditions are used for different target zeolites. For example, when the zeolite is Y zeolite, the hydrothermal crystallization temperature may be, for example, 80-120°C, preferably 95-105°C, and the hydrothermal crystallization time may be, for example, 20-60 hours, preferably 28-36 hours.

In a further preferred embodiment, the silicon source is selected from water glass, silica sol, sodium silicate, or combinations thereof; the alkali source is selected from alkali metal hydroxide, aqueous ammonia or combinations thereof; the structure directing agent or template agent is selected from tetramethyl ammonium bromide, tetraethyl ammonium bromide and hexamethylene imine.

In certain further preferred embodiments, the modified activated alumina substrate, the silicon source, the alkali source, the optional structure directing agent or template agent, and water are used with amounts calculated based on Na₂O, Al₂O₃, SiO₂, and H₂O, in a molar ratio of Na₂O: Al₂O₃: SiO₂: H₂O = 0.1-1.0: 1: 0.5-2.0: 10-50, wherein the obtained aluminosilicate zeolite is Y zeolite.

In certain further preferred embodiments, the modified activated alumina substrate, the silicon source, the alkali source, the optional structure directing agent or template agent, and water are used with amounts calculated based on Na₂O, Al₂O₃, SiO₂, and H₂O and the template agent calculated based on R, in a molar ratio of Na₂O: Al₂O₃: SiO₂: H₂O: R = 0.1-3.0: 1: 10.0-100.0: 300-800: 10-40, wherein the obtained aluminosilicate zeolite is MCM-22 zeolite.

In certain further preferred embodiments, the modified activated alumina substrate, the silicon source, the alkali source, the optional structure directing agent or template agent, and water are used with amounts calculated based on Na₂O, Al₂O₃, SiO₂, and H₂O and the template agent calculated based on R, in a molar ratio of Na₂O: Al₂O₃: SiO₂: H₂O: R = 0.01-2: 1: 20.0-100.0: 100-500: 5-30, wherein the obtained aluminosilicate zeolite is β zeolite.

In the present application, after hydrothermal crystallization, the aluminosilicate zeolite-hydrogenation metal component-alumina composite can be obtained by washing with deionized water and then drying. The drying can be carried out under normal pressure or reduced pressure, and the drying temperature can be 40-250°C, preferably 60-150°C; the drying time may be 8-36 hours, preferably 12-24 hours.

In the third aspect, the present application provides an aluminosilicate zeolite based catalyst comprising a binder and the aluminosilicate zeolite-hydrogenation metal component-alumina composite of the present application, wherein the catalyst has an aluminum content (calculated as alumina) of 10-85 wt%, preferably 15-80 wt%, a silicon content (calculated as silica) of 10-85 wt%, preferably 15-80 wt%, and a hydrogenation metal component content (calculated as metal) of 0.01-5 wt%, preferably 0.1-3 wt%, based on the total mass of the catalyst.

In the present application, there is no particular limitation on the binder, which may be those conventionally used for catalyst preparation in the art. In a preferred embodiment, the binder is an inorganic oxide, preferably selected from oxides of elements of Groups IIA, IVB, IIIA and IVA of the Periodic Table or combinations thereof, more preferably selected from alumina, silica, titania or combinations thereof. The use of the above-mentioned preferred binders is conducive to improving the overall performance of the catalyst.

According to the present application, there is no particular limitation on the shape of the aluminosilicate zeolite based catalyst, which may be in any physical form, such as powder, granule, or molded product, such as pallet, strip, or trilobe.

Without being bound to any specific theory, it is believed that in the aluminosilicate zeolite based catalyst of the present application, most of the hydrogenation-active metal is sandwiched between the aluminosilicate zeolite and the alumina, rather than being dispersed on the outer surface of the zeolite, which is more conducive to improving the coupling of hydrogenation and alkylation reactions, thereby improving the reactivity and selectivity of the catalyst in the hydroalkylation reaction of aromatics, particularly the hydroalkylation reaction of benzene to produce cyclohexylbenzene.

In the fourth aspect, there is provided a method for preparing an aluminosilicate zeolite based catalyst, comprising steps of:
I) providing an aluminosilicate zeolite-hydrogenation metal component-alumina composite according to the first aspect of the present application or preparing an aluminosilicate zeolite-hydrogenation metal component-alumina composite according to the method of the second aspect of the present application; and
II) kneading and molding the aluminosilicate zeolite-hydrogenation metal component-alumina composite with a binder source, followed by calcination and optionally activation, to obtain the catalyst.

In the present application, there is no particular limitation on the binder source, which may be those conventionally used for catalyst preparation in the art. In a preferred embodiment, the binder source is an inorganic oxide or those that can be converted into an inorganic oxide under the calcination conditions, and the inorganic oxide is preferably selected from oxides of elements from Groups IIA, IVB, IIIA and IVA of the Periodic Table or combinations thereof, more preferably from alumina, silica or combinations thereof. For example, the binder source may be alumina.

In step II), the kneading and molding may be performed by a conventional method in the art, such as extruding, but it is not strictly limited in the present application.

In a preferred embodiment, the resulting molded product is dried after kneading and molding. The drying may be carried out in a conventional manner in the art, and preferably the drying conditions include: a temperature of 40-250°C, preferably 60-150°C; a drying time of 8-30 hours, preferably 10-20 hours. The drying may be carried out under normal pressure or under reduced pressure.

In step II), the calcination may be carried out in a conventional manner in the art. In a preferred embodiment, the calcination conditions include: a calcination temperature of 300-650°C, preferably 400-600°C and/or a calcination time of 1-10 hours, preferably 3-6 hours. The calcination is carried out in an oxygen-containing atmosphere, such as air or an oxygen atmosphere.

In a preferred embodiment, the activation in step II) includes: subjecting the molded solid after calcination in step II) to ammonium exchange and reduction in sequence.

In a further preferred embodiment, conditions of the ammonium exchange include: a mass ratio of the molded solid to the ammonium salt to water of 1: 1-15: 1-15, an exchange temperature of 25-100°C, preferably 60-90°C, and an exchange time of 0.5-5 hours, preferably 1-2 hours. Further preferably, after the ammonium exchange, washing with deionized water and then drying are carried out. The drying can be carried out under normal pressure or reduced pressure, and the drying temperature may be 40-250°C, preferably 60-150°C; the drying time may be 8-36 hours, preferably 12-24 hours.

In a further preferred embodiment, conditions of the reduction include: carrying out the reduction under hydrogen atmosphere, at a reduction temperature of 100-500°C, preferably 190-400°C, for a reduction time of 0.5-12 hours, preferably 3-5 hours, and at a hydrogen volume space velocity of 100-600 h⁻¹, preferably 300-400 h⁻¹.

According to the present application, the aluminosilicate zeolite based catalyst may be prepared in any physical form, such as a powder, granule, or molded product, such as pallet, strip, or trilobe. There is no particular limitation on the physical form in the present application, which accordingly may be obtained in any conventional manner known in the art.

In the fifth aspect, there is provided an aluminosilicate zeolite based catalyst prepared according to the method of the present application for preparing an aluminosilicate zeolite based catalyst.

In the sixth aspect, there is provided use of the aluminosilicate zeolite based catalyst according to the third aspect or the fifth aspect of the present application in a hydroalkylation reaction of aromatics.

In the seventh aspect, the present application provides a method for hydroalkylation of aromatics, comprising contacting a feedstock of aromatics and an alkylating agent in the presence of hydrogen with the aluminosilicate zeolite based catalyst according to the third aspect or the fifth aspect of the present application to carry out a hydroalkylation reaction.

In a preferred embodiment, the feedstock of aromatics is selected from benzene, toluene, xylene, or combinations thereof.

In a preferred embodiment, the hydroalkylation reaction conditions include: a reaction temperature of 80-200°C, a reaction pressure of 0.1-2.0MPa, a molar ratio of hydrogen to benzene of 0.1-20.0, and a mass space velocity of benzene of 0.1-2.0 h⁻¹.

In the eighth aspect, there is provided a method for synthesizing cyclohexylbenzene, comprising step of: contacting and reacting benzene with the aluminosilicate zeolite based catalyst according to the third aspect or the fifth aspect of the present application in the presence of hydrogen to obtain cyclohexylbenzene.

In a preferred embodiment, conditions of the reaction include: a reaction temperature of 80-200°C, a reaction pressure of 0.1-2.0MPa, a molar ratio of hydrogen to benzene of 0.1-20.0, and a mass space velocity of benzene of 0.1-2.0 h⁻¹.

### Examples

The present application will be further described in detail with reference to examples below, but the present application is not limited thereto.

The instruments and test methods used in the following examples and Comparative Examples are as follows:
Thermo Fisher Nicolet IS10 infrared spectrometer was used to measure the pyridine infrared spectrum of the sample. During the measurement, the sample was dried under an infrared lamp for 30 minutes, and after being mixed with potassium bromide at a ratio of 1:50, it was pressed into a tablet for measurement. Before and after the measurement, the carbon dioxide background was subtracted.

The model of the X-ray fluorescence spectrometer (XRF) was Axios mAX model X-ray fluorescence spectrometer from Panalytical, Netherlands, and a standard-free quantitative method was used for quantitative analysis.

The model of the X-ray photoelectron spectroscopy (XPS) was K-Alpha model X-ray photoelectron spectrometer from Thermo Fisher, America, with an optimal resolution < 30 µm, an optimal energy resolution < 0.5 eV FWHM and a C1s energy resolution < 0.85 eV. The following conditions were used: Al Kα target (hv = 1486.68 eV), ion source energy range: 100 to 3 keV, maximum beam current: 4 µA, optimal vacuum of analysis chamber: 5×10⁻⁹ mbar. In the measurement, the surface was not subjected to sputtering treatment.

X-ray diffraction analysis (XRD): the samples were subjected to phase analysis using powder XRD method, and the relative crystallinity and the framework silicon to aluminum ratio were calculated. The instrument used was D/max-1400 model X-ray powder diffractometer from Rigaku, Japan. The following conditions were used: Cu-Ka radiation (wavelength: 1.541Å), tube current 40 mA, tube voltage 40 kV, and scanning angle range of phase analysis: 5-50 °.

In the following examples and Comparative Examples, the starting materials and reagents used were all commercially available materials with a purity of reagent-pure, unless otherwise specified.

In the following examples, the preparation of the aluminosilicate zeolite-hydrogenation metal component-alumina composite was as follows, but was not limited to the embodiments described below:
Y zeolite-hydrogenation metal component-alumina composite
   67.2 g of water glass was taken and charged into a beaker, and the temperature inside the beaker was heated to 50°C by using a water bath. 52 g of a directing agent was added, and the mixture was stirred uniformly. Thereafter, 12 g of sodium hydroxide was added to adjust the pH value of the system to above 10. Following thorough mixing to uniform, 88 g of water and 50 g of modified activated alumina substrate were added, and the mixture was stirred for 60 minutes. Afterwards, it was charged into a reaction kettle, and crystallized at 100°C for 20 hours, then the product was filtered, washed and dried at 120°C for 12 hours to obtain a sample of Y-hydrogenation metal component-alumina composite. Herein, the directing agent was prepared by stirring 55.8 g of water glass and 56.8 g of sodium aluminate solution at room temperature to react for 24 hours.
MCM-22 zeolite-hydrogenation metal component-alumina composite
   293 g of silica sol was taken and charged into a beaker, and the temperature inside the beaker was heated to 50°C by using a water bath. 144 g of hexamethylene amine template agent and 5.6 g of crystal seed were added, and the mixture was stirred uniformly. Thereafter, 10 g of sodium hydroxide was added to adjust the pH value of the system to above 10. Following thorough mixing to uniform, 970 g of water and 50 g of modified activated alumina substrate were added, and the mixture was stirred for 60 minutes. Afterwards, it was charged into a reaction kettle, and crystallized at 100°C for 28 hours, then the product was filtered, washed and dried at 120°C for 12 hours to obtain a sample of MCM-22-hydrogenation metal component-alumina composite.
β zeolite-hydrogenation metal component-alumina composite
   443 g of deionized water was taken and charged into a beaker, 32.6 g of sodium hydroxide was added to adjust the pH value of the system to above 10, and the mixture was continuously stirred to be completely dissolved. The temperature inside the beaker was heated to 50°C by using a water bath. 50 g of modified activated alumina substrate, 960 g of tetraethylammonium hydroxide and 520 g of tetraethylammonium bromide (TEA) were added and the mixture was stirred for 0.5 hour. Thereafter, 2445 g of 40% silica sol was added and the mixture was stirred continuously for 1 hour and charged into a crystallization kettle. The crystallization was carried out under stirring, at a crystallization temperature of 150°C, for a crystallization time of 40 hours, then the product was filtered, washed and dried at 120°C for 12 hours to obtain a sample of β-hydrogenation metal component-alumina composite.
Mixed zeolites-hydrogenation metal component-alumina composite
   The crystallization of the zeolites was carried out independently by using the methods for synthesizing zeolites as described above, and then the obtained crystallized products were mixed, thoroughly stirred, then filtered, washed and dried at 120°C for 12 hours to obtain the mixed zeolites-hydrogenation metal component-alumina composite.

### Example 1

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina.
(2) 96 g of the activated alumina was taken as a carrier and impregnated with 3 g of Ru in equal volume. The impregnated mixture was dried at 120°C for 12 hours and calcined at 550°C for 5 hours to obtain Ru/Al₂O₃.
(3) 50 g of Ru/Al₂O₃ was taken as a substrate to synthesize Y zeolite according to the method as described above, so as to obtain a Y-Ru/Al₂O₃ composite. The crystallinity and elemental analysis results of the composite are shown in Table 1.
(4) 50 g of the Y-Ru/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the resulting molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst A. Based on the charging amounts, Catalyst A contained 1.5 wt% of Ru, 50.5 wt% of the composite and 49.5 wt% of the binder, and the elemental analysis results thereof are shown in Table 2.

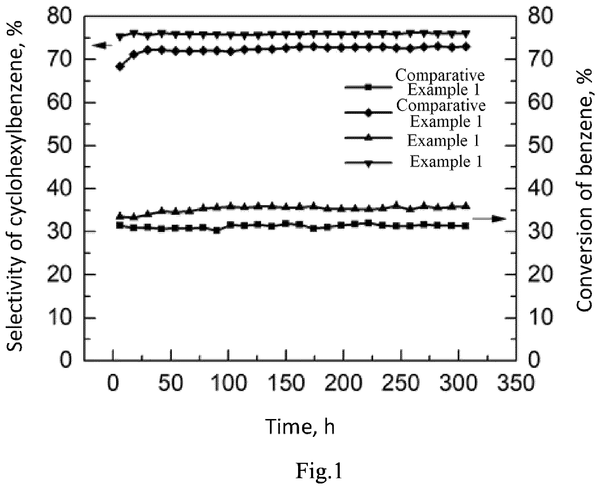

Catalyst A was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3, and the results of the long-term run are shown in FIG. 1.

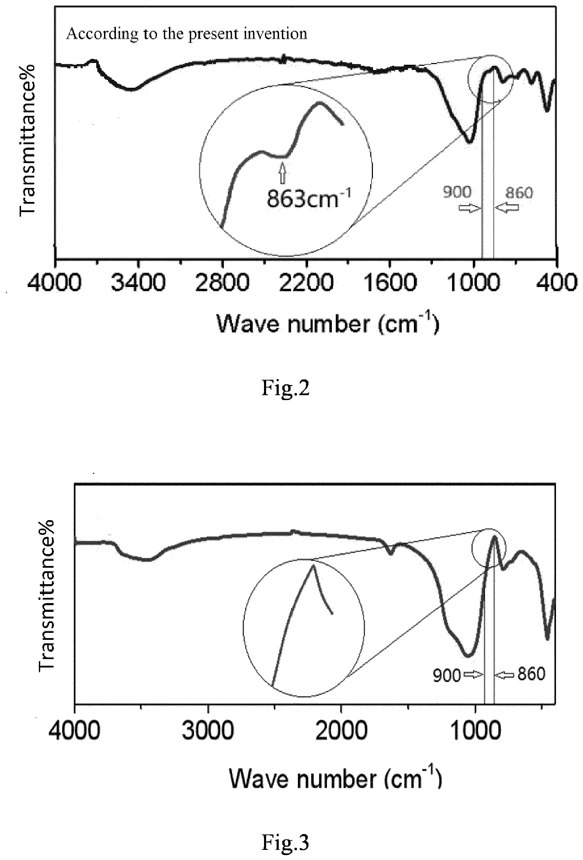

FIG. 2 shows the infrared spectrum of the Y-Ru/Al₂O₃ composite obtained in Example 1, which exhibits a characteristic peak at 863 cm⁻¹ within the range of 860-900 cm⁻¹, indicating the presence of chemical bonding between the zeolite and alumina.

### Example 2

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina;
(2) 99 g of the activated alumina was taken as a carrier and impregnated with 1 g of Pd in equal volume. The impregnated mixture was dried at 120°C for 12 hours and calcined at 550°C for 5 hours to obtain Pd/Al₂O₃;
(3) 50 g of Pd/Al₂O₃ was taken as a substrate to synthesize Y zeolite according to the method as described above, so as to obtain a Y-Pd/Al₂O₃ composite. The crystallinity and elemental analysis results of the composite are shown in Table 1;
(4) Then, 50 g of the Y-Pd/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst B. Based on the charging amounts, Catalyst B contained 0.5 wt% of Pd, 50.5 wt% of the composite and 49.5 wt% of the binder, and the elemental analysis results thereof are shown in Table 2.

Catalyst B was evaluated for the hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the Y-Pd/Al₂O₃ composite obtained in Example 2 is similar to that in Example 1.

### Example 3

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina;
(2) 95 g of the activated alumina was taken as a carrier and impregnated with 5 g of Ni in equal volume. The impregnated mixture was dried at 120°C for 12 hours and calcined at 550°C for 5 hours to obtain Ni/Al₂O₃;
(3) 50 g of Ni/Al₂O₃ was taken as a substrate to synthesize Y zeolite according to the method as described above, so as to obtain a Y-Ni/Al₂O₃ composite. The crystallinity and elemental analysis results of the composite are shown in Table 1;
(4) Then, 50 g of the Y-Ni/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst C. Based on the charging amounts, Catalyst C contained 2.5 wt% of Ni, 50.5 wt% of the composite and 49.5 wt% of the binder, and the elemental analysis results of the catalyst are shown in Table 2.

Catalyst C was evaluated for hydroalkylation reaction. The mass space velocity of benzene was 0.45 h⁻¹, the feed rate of benzene was 0.075 g/min, and the feed rate of hydrogen was 10.9 mL/min. The reaction temperature was 150°C and the reaction pressure was 0.12 MPa. The results of the reaction after 32 hours are shown in Table 3.

The infrared spectrum of the Y-Ni/Al₂O₃ composite obtained in Example 3 is similar to that in Example 1.

### Example 4

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina;
(2) 96 g of the activated alumina was taken as a carrier and impregnated with 3 g of Ru in equal volume. The impregnated mixture was dried at 120°C for 12 hours and calcined at 550°C for 5 hours to obtain Ru/Al₂O₃;
(3) 50 g of Ru/Al₂O₃ was taken as a substrate to synthesize β zeolite according to the method as described above, so as to obtain a β-Ru/Al₂O₃ composite. The crystallinity and elemental analysis results of the composite are shown in Table 1;
(4) Then, 50 g of the β-Ru/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst D. Based on the charging amounts, Catalyst D contained 1.5 wt% of Ru, 50.5 wt% of the composite and 49.5 wt% of the binder, and the elemental analysis results of the catalyst are shown in Table 2.

Catalyst D was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the β-Ru/Al₂O₃ composite obtained in Example 4 is similar to that in Example 1.

### Example 5

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina;
(2) 99 g of the activated alumina was taken as a carrier and impregnated with 1 g of Pd in equal volume. The impregnated mixture was dried at 120°C for 12 hours and calcined at 550°C for 5 hours to obtain Pd/Al₂O₃;
(3) 50 g of Pd/Al₂O₃ was taken as a substrate to synthesize β zeolite according to the method as described above, so as to obtain a β-Pd/Al₂O₃ composite. The crystallinity and elemental analysis results of the composite are shown in Table 1;
(4) Then, 50 g of the β-Pd/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst E. Based on the charging amounts, Catalyst E contained 0.5 wt% of Pd, 50.5 wt% of the composite and 49.5 wt% of the binder, and the elemental analysis results of the catalyst are shown in Table 2.

Catalyst E was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the β-Pd/Al₂O₃ composite obtained in Example 5 is similar to that in Example 1.

### Example 6

(1) 200 g of hydrated alumina was taken and calcined at 600°C for 5 hours. Then, 100 g of the calcined powder and 100 g of ammonium nitrate were taken and added into 500 g of deionized water, and the mixture was treated at 60°C for 2 hours. Thereafter, the product was washed with deionized water and dried at 120°C for 12 hours, followed by calcination at 550°C for 5 hours to obtain activated alumina;
(2) 95 g of the activated alumina was taken as a carrier and impregnated with 5 g of Ni in equal volume. The impregnated mixture was dried at 120°C for 12 hours and calcined at 550°C for 5 hours to obtain Ni/Al₂O₃;
(3) 50 g of Ni/Al₂O₃ was taken as a substrate to synthesize β zeolite according to the method as described above, so as to obtain a β-Ni/Al₂O₃ composite. The crystallinity and elemental analysis results of the composite are shown in Table 1;
(4) Then, 50 g of the β-Ni/Al₂O₃ composite was taken and mixed with 70 g of alumina. The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst F. Based on the charging amounts, Catalyst F contained 2.5 wt% of Ni, 50.5 wt% of the composite, and 49.5 wt% of the binder, and the elemental analysis results of the catalyst are shown in Table 2.

Catalyst F was evaluated for hydroalkylation reaction. The mass space velocity of benzene was 0.45 h⁻¹, the feed rate of benzene was 0.075 g/min, and the feed rate of hydrogen was 10.9 mL/min. The reaction temperature was 150°C and the reaction pressure was 0.12 MPa. The results of the reaction after 32 hours are shown in Table 3.

The infrared spectrum of the β-Ni/Al₂O₃ composite obtained in Example 6 is similar to that in Example 1.

### Example 7

(1) 200 g of hydrated alumina were taken and calcined at 600°C for 5 hours. Then 100 g of calcined powder and 100 g of ammonium nitrate were taken and added into 500 g of deionized water, and the mixture was treated at 60°C for 2 hours. Thereafter, the product was washed with deionized water and dried at 120°C for 12 hours, and then the ammonium zeolite was calcined at 550°C for 5 hours to obtain activated alumina;
(2) 96 g of the activated alumina was taken as a carrier and impregnated with 3 g of Ru in equal volume. The impregnated mixture was dried at 120°C for 12 hours and calcined at 550°C for 5 hours to obtain Ru/Al₂O₃;
(3) 50 g of Ru/Al₂O₃ was taken as a substrate to synthesize MCM-22 zeolite according to the method as described above, so as to obtain a MCM-22-Ru/Al₂O₃ composite. The crystallinity and elemental analysis results of the composite are shown in Table 1;
(4) Then, 50 g of the MCM-22-Ru/Al₂O₃ composite was taken and mixed with 70 g of a binder (alumina). The mixture was kneaded and molded into strips, dried at 120°C for 12 hours, and then calcined at 600°C for 5 hours to obtain molded solid; 30 g of the molded solid and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst G. Based on the charging amounts, Catalyst G contained 1.5 wt% of Ru, 50.5 wt% of the composite and 49.5 wt% of the binder, and the elemental analysis results of the catalyst are shown in Table 2.

Catalyst G was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the MCM-22-Ru/Al₂O₃ complex obtained in Example 7 is similar to that in Example 1.

### Example 8

A catalyst was prepared with reference to Example 1, step (4), except that: 25 g of the Y-Ru/Al₂O₃ composite obtained in step (3) of Example 1 and 25 g of the β-Ru/Al₂O₃ composite obtained in step (3) of Example 4 were used and mixed with 70 g of a binder (alumina), while other conditions remained the same, to obtain Catalyst H. Based on the charging amounts, Catalyst H contained 1.5 wt% of Ru, 50.5 wt% of the composite and 49.5 wt% of the binder, and the elemental analysis results of the catalyst are shown in Table 2.

Catalyst H was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

### Example 9

A catalyst was prepared with reference to Example 1, except that: in step (4), 50 g of the Y-Ru/Al₂O₃ composite was taken and mixed with 22.5 g of a binder (alumina), while other conditions remained the same, to obtain Catalyst I. Based on the charging amounts, Catalyst I contained 2.3 wt% of Ru, 76 wt% of the composite, and 24 wt% of the binder, and the elemental analysis results of the catalyst are shown in Table 2.

Catalyst I was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

### Example 10

A catalyst was prepared with reference to Example 1, except that: 70 g of porous silica gel was used as the binder, to obtain Catalyst J. Based on the charging amounts, Catalyst J contained 1.5 wt% of Ru, 43.4 wt% of the composite, and 56.5 wt% of the binder, and the elemental analysis results of the catalyst are shown in Table 2.

Catalyst J was evaluated for hydroalkylation reaction. The mass space velocity of benzene was 1.0 h⁻¹, the molar ratio of hydrogen to benzene was 2, the reaction temperature was 150°C, and the reaction pressure is 0.10 MPa. The results of the reaction after 32 hours are shown in Table 3.

**Table 1. Compositions and properties of the composites obtained in Examples 1-7**

| No. | Crystallinity, % | Sodium oxide content in wt% | Silica content in wt% | Alumina content in wt% | XPS Metal content in wt% | XRF Metal content in wt% | XPS metal content/XR F Metal content |
|---|---|---|---|---|---|---|---|
| Example 1 | 70 | 9.1 | 45.5 | 42.6 | 0.5 | 2.8 | 17.9% |
| Example 2 | 59 | 7.67 | 38.35 | 53.58 | 0.01 | 0.4 | 2.5% |
| Example 3 | 55 | 7.15 | 35.75 | 53.3 | 0.7 | 3.8 | 18.4% |
| Example 4 | 63 | 1.89 | 56.7 | 39.91 | 0.03 | 1.5 | 2% |
| Example 5 | 52 | 1.56 | 46.8 | 51.44 | 0.02 | 0.2 | 10% |
| Example 6 | 53 | 1.59 | 47.7 | 47.51 | 0.5 | 3.2 | 15.6% |
| Example 7 | 65 | 1.95 | 58.5 | 37.75 | 0.02 | 1.8 | 1.1% |

**Table 2. Compositions of catalysts obtained in Examples 1-10**

| No. | Catalyst | Silica content in wt% | Alumina content in wt% | Metal content in wt% |
|---|---|---|---|---|
| Example 1 | A | 26.1 | 72.4 | 1.5 |
| Example 2 | B | 21.4 | 78.3 | 0.3 |
| Example 3 | C | 20.3 | 78.0 | 1.7 |
| Example 4 | D | 23.4 | 75.0 | 1.6 |
| Example 5 | E | 19.6 | 79.9 | 0.5 |
| Example 6 | F | 20.0 | 78.0 | 2.0 |
| Example 7 | G | 24.3 | 74.3 | 1.5 |
| Example 8 | H | 25.2 | 70.7 | 4.1 |
| Example 9 | I | 39.2 | 59.2 | 1.6 |
| Example 10 | J | 78.3 | 19.8 | 1.9 |

### Comparative Example 1

98.5 g of Y zeolite was taken as a carrier and impregnated with 1.5 g of Ru in equal volume. The impregnated mixture was dried at 120°C for 12 hours and calcined at 550°C for 5 hours to obtain Ru/Y. Then 50 g of Ru/Y was taken and mixed with 70 g of hydrated alumina. The mixture was kneaded and molded into strips, dried at 120°C for 12 hours, and then calcined at 600°C for 5 hours to obtain a Y-Ru/Al₂O₃ composite. 30 g of the Y-Ru/Al₂O₃ composite and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst K.

Catalyst K was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3, and the results of the long-term run are shown in FIG. 1.

FIG. 3 shows the infrared spectrum of the Y-Ru/Al₂O₃ composite obtained in Comparative Example 1, which exhibits no characteristic peak within the range of 860-900 cm⁻¹, indicating that the zeolite and alumina were physically mixed and there is no chemical bonding between them.

### Comparative Example 2

99.5 g of Y zeolite was taken as a carrier and impregnated with 0.5 g of Pd in equal volume. The impregnated mixture was dried at 120°C for 12 hours and calcined at 550°C for 5 hours to obtain Pd/Y. Then 50 g of Pd/Y was taken and mixed with 70 g of hydrated alumina. The mixture was kneaded and molded into strips, dried at 120°C for 12 hours, and then calcined at 600°C for 5 hours to obtain Y-Pd/Al₂O₃ composite. 30 g of the Y-Pd/Al₂O₃ composite and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst L.

Catalyst L was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the Y-Pd/Al₂O₃ composite obtained in Comparative Example 2 exhibits no characteristic peak within the range of 860-900 cm⁻¹.

### Comparative Example 3

90 g of Y zeolite was taken as a carrier and impregnated with 10 g of Ni in equal volume. The impregnated mixture was dried at 120°C for 12 hours and calcined at 550°C for 5 hours to obtain obtain Ni/Y. Then 50 g of Ni/Y was taken and mixed with 70 g of hydrated alumina. The mixture was kneaded and molded into strips, dried at 120°C for 12 hours, and then calcined at 600°C for 5 hours to obtain Y-Ni/Al₂O₃ composite. 30 g of the Y-Ni/Al₂O₃ composite and 30 g of ammonium nitrate were taken and added into 300 g of deionized water, and the mixture was treated at 60°C for 2 hours. The product was then washed with deionized water and dried at 120°C for 12 hours to obtain an ammonium zeolite. The ammonium zeolite was calcined at 550°C for 5 hours, and the obtained sample was then reduced at 230°C for 3 hours at a hydrogen volume space velocity of 300 h⁻¹ to obtain Catalyst M.

Catalyst M was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

The infrared spectrum of the Y-Ni/Al₂O₃ composite obtained in Comparative Example 3 exhibits no characteristic peak within the range of 860-900 cm⁻¹.

### Comparative Example 4

(1) 50 g of modified alumina was taken and mixed with 8.1 g of RuCl₃ (37% Ru), 32.6 g of sodium hydroxide, 760 g of tetraethylammonium bromide and 1050 g of coarse-pore silica gel. The mixture was ground thoroughly for 8 minutes and placed into a reaction kettle for crystallization at 150°C for 40 hours to obtain Ru/zeolite-alumina mixture.
(2) Referring to step (4) of Example 1, 50 g of the Ru/zeolite-alumina mixture was taken and mixed with 70 g of a binder (alumina), while other conditions remained the same, to obtain Catalyst O.

Catalyst O was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

### Comparative Example 5

(1) A certain amount of metal salt (referring to Example 1) was dissolved in water to obtain an aqueous metal salt solution, and the aqueous metal salt solution and the activated alumina obtained in step (1) of Example 1 were used in place of the water and modified activated alumina substrate used in the method for synthesizing the Y zeolite-hydrogenated metal component-alumina composite as described above, respectively, to obtain a composite.
(2) Referring to step (4) of Example 1, 50 g of the composite obtained in step (1) taken and was mixed with 70 g of a binder (alumina), while other conditions remained the same, to obtain Catalyst P.

Catalyst P was evaluated for hydroalkylation reaction. The mass space velocity of benzene was 1.0 h⁻¹, the molar ratio of hydrogen to benzene was 2, the reaction temperature was 150°C, and the reaction pressure was 0.10 MPa. The results of the reaction after 32 hours are shown in Table 3.

**Table 3. Evaluation results of hydroalkylation reaction of catalysts obtained in each of Examples and Comparative Examples**

| No. | Conversion of benzene, % | Selectivity of cyclohexane, % | Yield of cyclohexane, % | Selectivity of cyclohexylbe nzene, % | Yield of cyclohexylbe nzene, % |
|---|---|---|---|---|---|
| Example 1 | 33.1 | 7.7 | 2.5 | 75.5 | 25.0 |
| Example 2 | 34.0 | 6.2 | 2.1 | 74.2 | 25.2 |
| Example 3 | 32.6 | 6.9 | 2.2 | 72.3 | 23.6 |
| Example 4 | 33.0 | 9.4 | 3.1 | 75.3 | 24.8 |
| Example 5 | 33.7 | 7.9 | 2.7 | 74.1 | 25.0 |
| Example 6 | 34.0 | 7.6 | 2.6 | 73.2 | 24.9 |
| Example 7 | 33.4 | 9.3 | 3.1 | 72.5 | 24.2 |
| Example 8 | 33.8 | 7.3 | 2.5 | 74.8 | 25.3 |
| Example 9 | 33.0 | 9.5 | 3.1 | 70.0 | 23.1 |
| Example 10 | 32.5 | 7.8 | 2.5 | 78.2 | 25.4 |
| Comparative Example 1 | 30.8 | 15.1 | 4.7 | 72.1 | 22.2 |
| Comparative Example 2 | 30.6 | 14.6 | 4.5 | 69.5 | 21.3 |
| Comparative Example 3 | 29.5 | 16.0 | 4.7 | 64.5 | 19.0 |
| Comparative Example 4 | 29.0 | 26.6 | 7.7 | 59.5 | 17.3 |
| Comparative Example 5 | 25.5 | 14.8 | 3.8 | 75.3 | 19.2 |

As can be seen from the data shown in Table 3, compared to the catalysts of Comparative Examples 1-5, the catalysts obtained in Examples 1-10 exhibit higher conversion of benzene, as well as higher yield and selectivity of cyclohexylbenzene as a main product, while the selectivity and yield of cyclohexane as a by-product are significantly decreased.

As can be seen from the long-term run results of the catalysts obtained in Example 1 of the present application and Comparative Example 1 shown in Fig. 1, the catalyst of the present application is able to maintain high activity without a decline during the long-term run. Meanwhile, compared to the catalyst of Comparative Example 1, the catalyst of the present application exhibits higher conversion of benzene and higher selectivity of cyclohexylbenzene as a main product.

The preferred embodiments of the present application have been described in detail above with reference to the accompanying drawings. However, the present application is not limited to the specific details of the above embodiments. Within the scope of the technical concept of the present application, various simple modifications may be made to the technical solution of the present application, and these simple modifications all fall within the protection scope of the present application.

Furthermore, it should be noted that the various specific technical features described in the above embodiments may be combined in any suitable manner without contradiction. In order to avoid unnecessary repetition, the present application does not otherwise elaborate on various possible combinations.

In addition, the various different embodiments of the present application may also be combined with each other in any manner, provided that they do not violate the spirit of the present application. Such combinations should also be regarded as the disclosure of the present application.

## Claims

1. A composite of aluminosilicate zeolite-hydrogenation metal component-alumina, comprising an aluminosilicate zeolite, alumina and a hydrogenation metal component, wherein at least portion of the aluminosilicate zeolite in the composite is chemically bonded to at least portion of the alumina, such that the composite has an infrared spectrum exhibiting a characteristic peak within a range of 860-900 cm⁻¹.

2. The composite according to claim 1, wherein:
the aluminosilicate zeolite is selected from zeolites having a ten- or twelve-membered ring pore structure, or combinations thereof, preferably from β, Y, MCM-22, PSH-3, SSZ-25, MCM-49, MCM-56 zeolites, or combinations thereof, and more preferably from β, Y, MCM-22 zeolites, or combinations thereof; and/or
the hydrogenation metal component comprises hydrogenation active metal(s) selected from metals of Group VIB and Group VIII of the Periodic Table, or combinations thereof, preferably from Ru, Pd, Pt, Ni, Co, Mo, W, or combinations thereof, and more preferably from Pd, Ru, Ni, or combinations thereof.

3. The composite according to claim 1 or 2, wherein the composite has a zeolite crystallinity of 50-75%, preferably 50-70%, as detected by XRD.

4. The composite according to any one of claims 1-3, wherein the composite has a silicon content (calculated as silica) of 5-65 wt%, preferably 15-50 wt%, an aluminum content (calculated as alumina) of 30-94.99 wt%, preferably 50-85% and a hydrogenation metal component content (calculated as metal) of 0.01-5 wt%, preferably 0.1-3 wt%, based on the total mass of the composite.

5. The composite according to any one of claims 1-4, wherein the surface hydrogenation metal percentage content of the composite as measured by XPS method accounts for 0.1-25%, preferably 5-20% of the total hydrogenation metal percentage content of the composite as measured by XRF method.

6. The composite according to any one of claims 1-5, prepared by a method comprising steps of:
1) subjecting hydrated alumina to a first calcination, a treatment with an aqueous ammonium salt solution, and a second calcination in sequence to obtain an activated alumina substrate;
2) loading a hydrogenation metal component on the activated alumina substrate to obtain a modified activated alumina substrate; and
3) using the modified activated alumina substrate as a solid aluminum source and synthesizing an aluminosilicate zeolite by hydrothermal crystallization to obtain the composite.

7. A method for preparing the composite of aluminosilicate zeolite-hydrogenation metal component-alumina as claimed in any one of claims 1-6, comprising steps of:
1) subjecting hydrated alumina to a first calcination, a treatment with an aqueous ammonium salt solution, and a second calcination in sequence to obtain an activated alumina substrate;
2) loading a hydrogenation metal component on the activated alumina substrate to obtain a modified activated alumina substrate; and
3) using the modified activated alumina substrate as a solid aluminum source and synthesizing an aluminosilicate zeolite by hydrothermal crystallization to obtain the composite.

8. The method according to claim 7, wherein in step 1):
conditions of the first calcination and the second calcination each independently include: a calcination temperature of 500-650°C and/or a calcination time of 0.5-5 hours; and/or
conditions of the treatment with an ammonium salt aqueous solution include: a mass ratio of the hydrated alumina (based on the weight after calcination at 550°C for 5 hours) to the ammonium salt to water of 1: 1-15: 1-15, preferably 1: 1-2: 1-5; an exchange temperature of 25-100°C, preferably 60-90°C; and/or an exchange time of 0.5-5 hours, preferably 1-2 hours.

9. The method according to claim 7 or 8, wherein:
the hydrogenation metal component comprises hydrogenation active metal(s) selected from metals of Group VIB and Group VIII of the Periodic Table, or combinations thereof, preferably from Ru, Pd, Pt, Ni, Co, Mo, W, or combinations thereof, and more preferably from Pd, Ru, Ni, or combinations thereof; and/or
the loading in step (2) is achieved by impregnation, preferably by equal-volume impregnation, preferably, the loading in step (2) is carried out by: contacting the activated alumina substrate with an aqueous solution containing a soluble salt of the hydrogenation active metal at a contact temperature of 0-50°C for a contact time of 0.5-12 hours.

10. The method according to any one of claims 7-9, wherein step (3) is carried out by: reacting the modified activated alumina substrate, a silicon source, an alkali source, optionally a structure directing agent or template agent, and water under hydrothermal crystallization conditions to obtain the composite;
preferably, the hydrothermal crystallization conditions include: a hydrothermal crystallization temperature of 80-250°C, preferably 100-180°C, and/or a hydrothermal crystallization time of 20-60 hours, preferably 28-48 hours.
further preferably, the silicon source is selected from water glass, silica sol, sodium silicate, or combinations thereof; the alkali source is selected from alkali metal hydroxide, aqueous ammonia or combinations thereof; the structure directing agent or template agent is selected from tetramethyl ammonium bromide, tetraethyl ammonium bromide and hexamethylene imine.

11. An aluminosilicate zeolite based catalyst comprising a binder and the composite of aluminosilicate zeolite-hydrogenation metal component-alumina according to any one of claims 1-6, wherein the catalyst has an aluminum content (calculated as alumina) of 10-85 wt%, preferably 15-80 wt%, a silicon content (calculated as silica) of 10-85 wt%, preferably 15-80 wt%, and a hydrogenation metal component content (calculated as metal) of 0.01-5 wt%, preferably 0.1-3 wt%, based on the total mass of the catalyst.

12. The catalyst according to claim 11, wherein the binder is an inorganic oxide, preferably selected from oxides of elements of Groups IIA, IVB, IIIA and IVA of the Periodic Table or combinations thereof, more preferably selected from alumina, silica, titania or combinations thereof.

13. A method for preparing an aluminosilicate zeolite based catalyst, comprising steps of:
I) providing the composite of aluminosilicate zeolite-hydrogenation metal component-alumina as claimed in any one of claims 1-6 or preparing the composite of aluminosilicate zeolite-hydrogenation metal component-alumina according to the method as claimed in any one of claims 7-10; and
II) kneading and molding the composite of aluminosilicate zeolite-hydrogenation metal component-alumina with a binder source, followed by calcination and optionally activation, to obtain the catalyst.

14. The method according to claim 13, wherein:
conditions of the calcination in step II) include: a calcination temperature of 300-650°C, and/or a calcination time of 1-10 hours; and/or
the activation in step II) includes: subjecting the molded solid after calcination in step II) to ammonium exchange and reduction in sequence, preferably, conditions of the ammonium exchange include: a mass ratio of the molded solid to the ammonium salt to water of 1: 1-15: 1-15, an exchange temperature of 25-100°C, and an exchange time of 0.5-5 hours; and/or conditions of the reduction include: carrying out the reduction under hydrogen atmosphere, at a reduction temperature of 100-500°C, for a reduction time of 0.5-12 hours, and at a hydrogen volume space velocity of 100-600 h⁻¹.

15. An aluminosilicate zeolite based catalyst prepared by the method according to any one of claims 13-14.

16. Use of the aluminosilicate zeolite based catalyst as claimed in any one of claims 11-12 and 15 in a hydroalkylation reaction of aromatics, comprising contacting and reacting a feedstock of aromatics and an alkylating agent in the presence of hydrogen with the aluminosilicate zeolite based catalyst.

17. A method for synthesizing cyclohexylbenzene, comprising step of:
contacting and reacting benzene with the aluminosilicate zeolite based catalyst as claimed in any one of claims 11-12 and 15 in the presence of hydrogen to obtain cyclohexylbenzene;
preferably, conditions of the reaction include: a reaction temperature of 80-200°C, a reaction pressure of 0.1-2.0MPa, a molar ratio of hydrogen to benzene of 0.1-20.0, and a mass space velocity of benzene of 0.1-2.0 h⁻¹.
